Europäisches Patentamt

European Patent Office

Office européen des brevets

(11) Veröffentlichungsnummer: **0 335 290**
**A2**

(12) **EUROPÄISCHE PATENTANMELDUNG**

(21) Anmeldenummer: 89105350.6

(22) Anmeldetag: 25.03.89

(51) Int. Cl.⁴: **C07C 45/82 , C07C 45/84 , C07C 47/198 , C07C 29/80 , C07C 31/12**

(30) Priorität: 31.03.88 DE 3811059

(43) Veröffentlichungstag der Anmeldung: 04.10.89 Patentblatt 89/40

(84) Benannte Vertragsstaaten: BE CH DE ES FR GB IT LI NL SE

(71) Anmelder: HOECHST AKTIENGESELLSCHAFT Postfach 80 03 20 D-6230 Frankfurt am Main 80(DE)

(72) Erfinder: Driscoll, Robert Kenneth, Dr. Heilmann-Strasse 43 D-6000 Frankfurt am Main 50(DE) Erfinder: Leupold, Ingo, Dr. Am Zäunefeld 15 D-6392 Neu-Anspach(DE) Erfinder: Schönwälder, Karl-Heinz, Dr. Am Feldbergblick 6 D-6233 Kelkheim (Taunus)(DE)

(54) **Verfahren zur Trennung von Butanol und Butoxyacetaldehyd.**

(57) Verfahren zur Trennung von Butanol und Butoxyacetaldehyd, dadurch gekennzeichnet, daß Gemische, die diese enthalten, einer Destillation bei einem Druck von höchstens 660 mbar unterworfen werden.

Nach einer besonderen Ausführungsform werden Ausgangsgemische verwendet, die im wesentlichen Wasser, Butoxyacetaldehyd, Butanol und Butylglykol und gegebenenfalls Butyraldehyd enthalten.

EP 0 335 290 A2

## Verfahren zur Trennung von Butanol und Butoxyacetaldehyd

Die Erfindung betrifft ein Verfahren zur Trennung von Butanol und Butoxyacetaldehyd.

Alkoxyacetaldehyde sind wertvolle Ausgangsstoffe für die Herstellung von Farbstoffen, Kunststoffen, Riechstoffen, Harzen und Elastomeren. Ihre Herstellung durch Dehydrierung oder Oxydehydrierung von Äthylenglykolmonoalkyläthern (Alkylglykolen) ist oftmals in der Literatur beschrieben worden, z.B. von Drake et al., Journ. Am. Chem. Soc. 60 (1938), 73-76 und Keiko et al., Prikl. Zh. Chim. (Leningrad) 43 - (1970), 1137-40. Es ist allgemein bekannt, daß diese Verbindungen sich nur schwer selektiv oxydehydrieren lassen (Houben-Weyl 7/1 (1954) , S. 166 und 167). Eine bevorzugte Nebenreaktion ist die Abspaltung der Alkoxygruppe in Form des entsprechenden Alkohols.

Drake et al. beschreiben die qualitative Isolierung von Methoxy- und Äthoxyacetaldehyd als solchen bzw. als Azeotrop mit Wasser. Ein Großteil der erwünschten Produkte konnte von ihnen jedoch nicht isoliert werden, sondern war zu hochsiedenden Polymeren polymerisiert. Es wird dort außerdem erwähnt, daß die Isolierung von n-Butoxyacetaldehyd noch schwieriger ist. Keiko et al. beschreiben ebenfalls eine qualitative Abtrennung von Alkoxyacetaldehyden, u.a. Butoxyacetaldehyd, aus derartigen Reaktionsgemischen, die zwangsläufig noch Wasser enthalten. Angaben über die Isolierungsausbeuten von Butoxyacetaldehyd werden jedoch nicht gemacht.

In den US-PS 3 481 837 und 3 505 407 werden Verfahren zur Isolierung von Methoxyacetaldehyd aus dem Reaktionsprodukt der Oxydation von Methylglykol beschrieben. Es wurde dort festgestellt, daß eine vollständige destillative Trennung von Methanol und Methoxyacetaldehyd trotz ausreichendem Siedepunktsunterschied kaum möglich ist. Methanol und Wasser konnten jedoch von Methoxyacetaldehyd durch Zugabe eines bestimmten Schleppmittels (Acetonitril oder Chloroform) und anschließende azeotrope Destillation abgetrennt werden, so daß Methoxyacetaldehyd in einer Ausbeute vom 76 % mit mehr als 95 %iger Reinheit erhalten wurde. Der restliche Methoxyacetaldehyd ging als hochsiedendes Polymeres verloren. Die Zugabe eines Schleppmittels erfordert jedoch, zusätzliche Trennstufen durchzuführen, um Methoxyacetaldehyd vom Schleppmittel zu trennen und um reines Schleppmittel in das Verfahren zurückführen zu können.

Ein detailliertes Verfahren zur Isolierung von Butoxyacetaldehyd wurde bisher nicht beschrieben.

Es wurde nun überraschend gefunden, daß Butanol und Butoxyacetaldehyd sich in einem bestimmten Druckbereich, und zwar bei einem Druck von höchstens 660 mbar, gut voneinander destillativ trennen lassen. Die Verwendung eines zusätzlichen Schleppmittels ist nicht erforderlich.

Die Erfindung ermöglicht es auch, Butoxyacetaldehyd aus komplizierter zusammengesetzten Gemischen, die noch Butanol enthalten, zu isolieren. So gelingt es mit Hilfe des erfindungsgemäßen Verfahrens, den Butoxyacetaldehyd aus Gemischen zu isolieren, die neben Butanol noch Wasser enthalten. Dies wird dadurch ermöglicht, daß, wie ebenfalls gefunden wurde, n-Butoxyacetaldehyd mit Wasser ein leichter siedendes Azeotrop bildet, das nach der Kondensation in zwei Phasen zerfällt. Wenn man die obere, organische Phase in die Destillation zurückführt, kann man das Wasser auf diese Weise abtrennen, was eine große Vereinfachung bedeutet.

Es können jedoch auch noch komplizierter zusammengesetzte Gemische mit Hilfe des erfindungsgemäßen Verfahrens getrennt werden, z.B. solche, die im wesentlichen Wasser, Butoxyacetaldehyd, Butanol und Butylglykol sowie gegebenenfalls weitere Stoffe, wie Butyraldehyd enthalten. Derartige Gemische fallen beispielsweise bei der katalytischen Oxydehydrierung von Butylglykol, wie n-Butylglykol, in der Gasphase an. Zur Trennung solcher Gemische geht man erfindungsgemäß vorteilhaft so vor, daß man

a) in einer ersten Stufe das Wasser destillativ als Azeotrop mit dem Butoxyacetaldehyd über Kopf destilliert, das kondensierte Destillat in zwei Phasen zerfallen läßt und die obere, organische Phase in die Destillation zurückführt,

b) in einer zweiten Stufe das Sumpfprodukt a) bei einem Druck von höchstens 660 mbar destilliert, wobei Butanol und restliche Leichtsieder über Kopf entfernt werden und im wesentlichen ein Sumpf aus Butoxyacetaldehyd und Butylglykol erhalten wird, und

c) in einer dritten Stufe aus dem Sumpfprodukt von b) fast reinen Butoxyacetaldehyd über Kopf abdestilliert, so daß Butylglykol im Sumpf zurückbleibt.

Die erste Stufe wird im allgemeinen im Bereich von 10 bis 2000 mbar durchgeführt, vorzugsweise von 50 bis 660 mbar. Geeignet ist z.B. eine Kolonne, für die nur wenige theoretische Böden benötigt werden. Dabei wird vorwiegend Wasser und etwa vorhandener Butyraldehyd und Butanol entfernt, die bekanntlich mit Wasser Azeotrope bilden. Da Butoxyacetaldehyd mit Wasser ebenfalls ein Azeotrop bildet, kommen bei der Destillation Gemische aus Butanol, Butyraldehyd, Wasser und beträchtlichen Teilen Butoxyacetaldehyd

2

über Kopf. Die genannten drei organischen Stoffe sind jedoch in flüssigem Zustand nur begrenzt mit Wasser mischbar, so daß das Destillat in zwei Phasen zerfällt. So enthält die untere Phase bei 20° C etwa 90 Gew.-% Wasser, während die obere, organische Phase etwa 11 Gew.-% Wasser enthält. Etwa 90 % des im Destillat befindlichen Butoxyacetaldehyds werden in der oberen Phase wiedergefunden. Diese wird in die Destillation zurückgeführt, d.h. sie wird verwendet, um das Wasser auszuschleppen.

Sehr wenig (etwa 2 %) des im Ausgangsgemisch enthaltenen Butoxyacetaldehyds geht in der wäßrigen Phase verloren. Falls erwünscht, können diese geringen Verluste durch Extraktion der wäßrigen Phase mit üblichen Extraktionsmitteln, z.B. Butyraldehyd oder Butanol, vermieden werden. Anschließend muß der Butoxyacetaldehyd vom Extraktionsmittel destillativ getrennt werden, was im Normalfall viel Aufwand für wenig Produkt bedeuten würde.

Das Eingangsmaterial für die zweite Stufe enthält im wesentlichen Butanol, Butoxyacetaldehyd, Butylglykol und Butyraldehyd. In dieser wird Butanol weitgehend von Butoxyacetaldehyd getrennt. Hierfür ist die Einhaltung eines Druckes von höchstens 660 mbar notwendig. Im allgemeinen beträgt der Druck mindestens 16 mbar.

Das komplizierte Destillationsverhalten von Gemischen von Butanol und Butoxyacetaldehyd wird z.B. daraus ersichtlich, daß aus solchen Gemischen, die nur einen relativ kleinen Anteil an n-Butanol enthalten, bei 16 mbar zunächst reiner Butoxyacetaldehyd und anschließend ein Gemisch aus Butanol und Butoxyacetaldehyd abdestilliert wird. Der nach der Destillation erhaltene Sumpf enthält immer noch viel Butanol. Es ist also bei 16 mbar zwar möglich, Butanol und Butoxyacetaldehyd teilweise voneinander zu trennen, aber nicht vollständig. Destilliert man dasselbe Gemisch bei 395 mbar, so erhält man zunächst reines n-Butanol und anschließend Butoxyacetaldehyd, wie aus den Siedepunkten (Kp 94° C bzw. 117° C) zu erwarten war. Die beiden Substanzen gehen also in umgekehrter Reihenfolge wie bei 16 mbar über. Zwar ist bei dem Druck von 395 mbar die destillative Trennung von Butanol und Butoxyacetaldehyd gut, jedoch besteht die Gefahr, daß sich ein Teil des Butoxyacetaldehyds zu hochsiedenden Polymeren zersetzt. Diese bei höheren Destillationsdrucken erfolgende Zersetzung kann jedoch durch die Anwendung einer kontinuierlichen Arbeitsweise vermindert werden, da dann die Verweilzeit des Butoxyacetaldehyds im Sumpf geringer ist.

Aus den vorgenannten Gründen wird die Destillation zweckmäßig im Bereich von 16 bis 395 mbar, bevorzugt von 40 bis 260 mbar und insbesondere von 65 bis 200 mbar durchgeführt.

Bei der Trennung der vorgenannten kompliziert zusammengesetzten Gemische besteht das Eingangsmaterial für die dritte Stufe im wesentlichen aus Butoxyacetaldehyd und Butylglykol. In dieser Stufe wird fast reiner Butoxyacetaldehyd am Kopf gewonnen, während Butylglykol im Sumpf anfällt. Das Butylglykol kann wieder im Oxydationsreaktor verwendet werden. Diese Destillation wird im allgemeinen im Bereich von 2 bis 1000 mbar durchgeführt, vorzugsweise von 10 bis 260 mbar. Besonders bevorzugt werden jedoch 10 bis 160 mbar.

Die drei Stufen können sowohl ansatzweise als auch kontinuierlich, z.B. unter Anwendung von Destillationskolonnen, durchgeführt werden. Eine kontinuierliche Arbeitsweise, mindestens für die Stufen b) und c) wird bevorzugt. Eine solche Arbeitsweise ist besonders dann zu empfehlen, wenn bei relativ hohen Drucken destilliert wird.

Wenn die erste Stufe ansatzweise durchgeführt wird, kann die organische Phase am Kopf zurückgeführt werden, bis sich keine wäßrige Phase mehr bildet; anschließend kann der leichtsiedende Butyraldehyd abdestilliert werden. Arbeitet man dagegen in der ersten Stufe kontinuierlich, so ist zu empfehlen, den Butyraldehyd und etwa noch vorhandenes restliches Wasser zusammen mit Butanol erst in der zweiten Stufe über Kopf zu entfernen.

Nach einer anderen Ausführungsform kann man Gemische, die Butanol, Butoxyacetaldehyd und Butylglykol und gegebenenfalls weitere Substanzen wie Wasser und Butyraldehyd enthalten, in der Weise trennen, daß man zunächst die leichter siedenden Stoffe destillativ von dem am höchsten siedenden Butylglykol abtrennt, das also als Sumpf zurückbleibt, und das Destillat dann gemäß der für die Stufen a) und b) angegebenen Arbeitsweise aufarbeitet. Diese Ausführungsform ist jedoch weniger bevorzugt.

Etwa vorhandener Butyraldehyd wird regelmäßig mit dem Butanol ausgetragen.

Die Butylgruppen in Butylglykol, Butanol und Butoxyacetaldehyd können primär, also normal oder iso-, sekundär oder tertiär sein. Vorzugsweise sind sie jedoch alle n-Butylgruppen.

Im folgenden beziehen sich die Prozentangaben auf das Gewicht. Als Ausgangsmaterial wird in den Beispielen ein Rohprodukt verwendet, das mit Hilfe eines Silberkatalysators auf einem Aluminiumsilikatträger wie folgt hergestellt wurde:

200 g/h n-Butylglykol wurden zusammen mit 3980 Nl/h $N_2$ und 320 Nl/h Luft einem Verdampfer bei 300° C zudosiert. Anschließend wurde dieses Gemisch in einem Vorheizer auf 420° C gebracht und dem Reaktor zugeführt. Die maximale Reaktionstemperatur wurde mit einem beweglichen Thermoelement gemessen und betrug 420° C. Die Verweilzeit im Reaktor betrug etwa 0,27 Sekunden. Am Reaktorausgang

EP 0 335 290 A2

wurde das gasförmige Reaktionsprodukt zunächst mit einem Wasserkühler und anschließend mit einem Solekühler (T = -10° C) abgekühlt. Dabei fielen 201,2 g/h Kondensat mit folgender Zusammensetzung an: 77,2 % n-Butoxyacetaldehyd, 5,0 % n-Butylglykol, 3,1 % n-Butanol, 1,0 % n-Butyraldehyd und 11,7 % Wasser. CO, $CO_2$ und etwas Wasser und Butanol wurden nicht kondensiert. Die Ausbeute betrug 79 % bei 95 %igem Umsatz.

**Beispiele**

1) 1. Stufe - 3 kg des Kondensatgemisches wurden in einem 6 l-Kolben mit angeschlossenem Wasserabscheider bei 100 mbar erhitzt. Die Kopftemperatur stieg schnell auf 45° C an, was etwa dem Siedepunkt von Wasser bei diesem Druck entspricht. Das Destillat enthielt jedoch nur etwa 50 % Wasser und außerdem n-Butoxyacetaldehyd, n-Butyraldehyd, n-Butanol und wenig n-Butylglykol, d.h. das Destillat war ein Azeotrop mit mehreren Komponenten. Nach der Kondensation zerfiel das Destillat in eine obere, organische Phase, die wenig Wasser enthielt und eine untere Phase, die zu etwa 89 % aus Wasser bestand. Die obere Phase wurde kontinuierlich während der Detillation in den Kolben zurückgeführt. Die Destillation wurde fortgesetzt, bis das kondensierte Destillat nur aus einer Phase bestand und die Kopftemperatur auf 60° C angestiegen war. Dieses einphasige Destillat wurde schließlich dem Destillationssumpf zugegeben.

Während der Destillation wurden 384 g wäßrige Phase mit folgender Zusammensetzung entfernt: 88,8 % Wasser, 7,8 % n-Butoxyacetaldehyd, 1,3 % n-Butanol, 0,8 % n-Butyraldehyd und 1,3 % Sonstiges. Es wurden 2602 g Sumpf mit folgender Zusammensetzung erhalten: 87,9 % n-Butoxyacetaldehyd, 5,8 % n-Butylglykol, 3,4 % n-Butanol, 0,6 % n-Butyraldehyd, 0,3 % Wasser und 2,1 % Sonstiges. Somit wurden mehr als 97 % des Wassers aus dem Ausgangsmaterial entfernt, wobei nur etwa 1,3 % des darin enthaltenen n-Butoxyacetaldehyds mit der wäßrigen Phase verloren gingen.

2. Stufe - 100 g/h des zuvor erhaltenen Sumpfproduktes wurden auf 83° C vorgeheizt und in eine Silbermantel-Destillationskolonne von 2,5 cm Durchmesser am unteren Drittel eingespeist, die eine 120 cm hohe Schicht aus Braunschweiger Glaswendeln enthielt. Die Destillation wurde bei 132 mbar durchgeführt; das Rücklaufverhältnis betrug 14 : 1. Am Kopf wurde eine Fraktion mit Siedepunkt 70° C gewonnen, die n-Butanol und sonstige Leichtsieder enthielt. Dem Sumpf wurden 95,9 g/h eines Produktes der Zusammensetzung 90,2 % n-Butoxyacetaldehyd, 5,9 % n-Butylglykol, 0,4 % n-Butanol und 3,4 % Sonstiges entzogen.

3. Stufe - Das in der zweiten Stufe erhaltene Produkt wurde bei 78° C in eine zweite Destillationskolonne am oberen Drittel eingespeist. Diese Kolonne, gleichfalls mit Silbermantel, hatte denselben Durchmesser und enthielt ebenfalls eine 120 cm hohe Schicht aus Braunschweiger Glaswendeln. Die Destillation wurde bei 66 mbar durchgeführt; das Rücklaufverhältnis betrug 3 : 1. Es wurden 85,2 g/h Destillat mit einem Siedepunkt 71° C gewonnen, das am Kopf aus 99,5 % n-Butoxyacetaldehyd und 0,5 % n-Butanol bestand. Im Sumpf fiel ein Gemisch aus n-Butylglykol und sonstigen Hochsiedern an.

Gemäß diesem Beispiel konnten mehr als 95 % des im Oxydationsprodukt enthaltenen n-Butoxyacetaldehyds mit einer 99,5 %igen Reinheit gewonnen werden.

Frisch destillierter n-Butoxyacetaldehyd hat eine leuchtend grünliche Farbe. Nach einigen Stunden verliert die Flüssigkeit ihre Farbe und der Aldehyd liegt vorwiegend in Form von Oligomeren vor. Daraus kann der monomere Aldehyd problemlos wiedergewonnen werden, z.B. durch einfache Destillation unter vermindertem Druck.

2) 500 g des aus der ersten Stufe von Beispiel 1 erhaltenen Sumpfproduktes wurden diskontinuierlich in einer Silbermantelkolonne (Länge 120 cm, Durchmesser 2,5 cm, Füllung: Braunschweiger Glaswendeln, Rücklaufverhältnis 6 :1) bei 132 mbar destilliert. Nach Entfernung des restlichen n-Butyraldehyds und Wassers stieg die Kopftemperatur auf 70° C, entsprechend dem Siedepunkt von n-Butanol bei diesem Druck. Die Destillation wurde fortgeführt, bis die Kopftemperatur deutlich anstieg. Am Ende wurden 479 g Produkt der folgenden Zusammensetzung im Sumpf gefunden: 89,0 % n-Butoxyacetaldehyd, 5,9 % n-Butylglykol, 0,4 % n-Butanol und 4,6 % Sonstiges. Etwa 90 % des im Eingangsmaterial enthaltenen n-Butanols wurden über Kopf entfernt; 97 % des n-Butoxyacetaldehyds wurden wiedergefunden. Dieses Produkt wurde weiter verarbeitet, wie es im Beispiel 1 in der dritten Stufe beschrieben ist.

3) Beispiel 2 wurde mit weiteren 500 g des in der ersten Stufe von Beispiel 1 erhaltenen Sumpfprodukts wiederholt. Die Destillation wurde jedoch bei 395 mbar durchgeführt. Nach Entfernung des restlichen n-Butyraldehyds und Wassers stieg die Kopftemperatur auf 94° C entsprechend dem Siedepunkt von n-Butanol bei diesem Druck. Am Ende des Versuches enthielt der Sumpf 477 g eines Gemisches der Zusammensetzung: 69,1 % n-Butoxyacetaldehyd, 4,9 % n-Butylglykol, 0,4 % n-Butanol und 25,6 % Sonstiges. Hier wurden ebenfalls etwa 90 % des im Einsatz enthaltenen n-Butanols entfernt. Etwa 25 % des

4

n-Butoxyacetaldehyds haben sich aber zersetzt, wobei hochsiedendes Produkt im Sumpf entstand.

4) Beispiel 2 wurde mit weiteren 500 g des in der ersten Stufe von Beispiel 1 erhaltenen Sumpfproduktes wiederholt. Hier wurde jedoch die Destillation bei 16 mbar durchgeführt. Der Verlauf der Destillation ist in der folgenden Tabelle wiedergegeben:

|                     | Einsatz  | Frakt. 1 | Frakt. 2 | Frakt.3 | Sumpf |
|---------------------|----------|----------|----------|---------|-------|
| n-Butoxyacetaldehyd | 439,5 g  | 238,2    | 67,3     | 48,9    | 71,9  |
| n-Butanol           | 17       | 2,4      | 2,4      | 3,2     | 8,9   |
| n-Butylglykol       | 29       | -        | -        | -       | 28,3  |

Nach Entfernung des restlichen n-Butyraldehyds und Wassers stieg die Kopftemperatur gleich auf 44°C, was dem Siedepunkt von n-Butoxyacetaldehyd etwa entspricht. Reines n-Butanol siedet bei 33°C. Zunächst wurde eine Fraktion fast reinen n-Butoxyacetaldehyds am Kopf gewonnen. Weitere Fraktionen enthielten zunehmende Mengen an n-Butanol, die Kopftemperatur blieb jedoch fast konstant. Der Sumpf enthielt ebenfalls viel n-Butanol. n-Butanol und n-Butoxyacetaldehyd können also durch einfache Destillation bei 16 mbar zwar teilweise, aber nicht quantitativ voneinander getrennt werden. Es wurden etwa 97 % des n-Butoxyacetaldehyds wiedergefunden.

**Ansprüche**

1. Verfahren zur Trennung von Butanol und Butoxyacetaldehyd, dadurch gekennzeichnet, daß Gemische, die diese enthalten, einer Destillation bei einem Druck von höchstens 660 mbar unterworfen werden.

2. Verfahren nach Anspruch 1, dadurch gekennzeichnet, daß man die Destillation bei einem Druck von mindestens 16 mbar, vorzugsweise von 40 bis 260 mbar und insbesondere im Bereich von 65 bis 200 mbar durchführt.

3. Verfahren nach Anspruch 1 oder 2, dadurch gekennzeichnet, daß Ausgangsgemische verwendet werden, die im wesentlichen Wasser, Butanol und Butoxyacetaldehyd enthalten.

4. Verfahren nach einem oder mehreren der Ansprüche 1 bis 3, dadurch gekennzeichnet, daß Ausgangsgemische verwendet werden, die außerdem Butylglykol enthalten.

5. Verfahren nach einem oder mehreren der Ansprüche 1 bis 4, dadurch gekennzeichnet, daß Ausgangsgemische verwendet werden, die im wesentlichen Wasser, Butoxyacetaldehyd, Butanol und Butylglykol und gegebenenfalls Butyraldehyd enthalten.

6. Verfahren nach Anspruch 5, dadurch gekennzeichnet, daß man

a) in einer ersten Stufe das Wasser destillativ als Azeotrop mit dem Butoxyacetaldehyd über Kopf destilliert, das kondensierte Destillat in zwei Phasen zerfallen läßt und die obere, organische Phase in die Destillation zurückführt,

b) in einer zweiten Stufe das Sumpfprodukt a) bei einem Druck von höchstens 660 mbar destilliert, wobei Butanol und restliche Leichtsieder über Kopf entfernt werden und im wesentlichen ein Sumpf aus Butoxyacetaldehyd und Butylglykol erhalten wird, und

c) in einer dritten Stufe aus dem Sumpfprodukt von b) fast reinen Butoxyacetaldehyd über Kopf abdestilliert, so daß Butylglykol im Sumpf zurückbleibt.

7. Verfahren nach Anspruch 6, dadurch gekennzeichnet, daß die Destillation in der ersten Stufe a) im Bereich von 10 bis 2000, vorzugsweise von 50 bis 660 mbar, in der zweiten Stufe b) zweckmäßig im Bereich von 16 bis 395, vorzugsweise von 40 bis 260 und insbesondere von 65 bis 200 mbar und in der dritten Stufe c) im Bereich von 2 bis 1000, vorzugsweise von 10 bis 260 und insbesondere von 10 bis 160 mbar durchgeführt wird.

8. Verfahren nach Anspruch 4, dadurch gekennzeichnet, daß man Gemische einer Trennung unterwirft, die Butanol, Butoxyacetaldehyd, Butylglykol und gegebenenfalls weitere Substanzen enthalten, indem man zunächst die leichter siedenden Stoffe destillativ vom Butylglykol abtrennt und das Destillat je nach Zusammensetzung nach der Arbeitsweise gemäß den Ansprüchen 1, 2 oder den Stufen a) und b) gemäß den Ansprüchen 6 oder 7 aufarbeitet.